# EUROPEAN PATENT SPECIFICATION

(11) **EP 1 260 235 B1**
(45) Date of publication and mention of the grant of the patent: **16.02.2005**
(21) Application number: 02253657.7
(22) Date of filing: 23.05.2002
(51) Int. Cl.: A61L 2/16, A61L 2/26

(54) **Adjustable accumulator**
Einstellbarer Speicherbehälter
Accumulateur réglable

(30) Priority: 24.05.2001 US 864470
(43) Date of publication of application: 27.11.2002
(73) Proprietor: ETHICON, INC., Somerville, NJ 08876 (US)
(72) Inventor: Lukasik, Robert G., Lake Elsinore, CA 92530 (US); Lin, Szu-Min, Laguna Hills, CA 92653 (US)
(74) Representative: Fisher, Adrian John

(56) References cited:
- EP-A- 0 098 419
- DE-A- 2 231 082
- US-A- 5 527 507
- US-A- 5 951 948

## Description

### Background of the Invention

### Field of the Invention

This invention relates to an adjustable accumulator for delivering gas, liquid, or solids, for use, for example, in sterilization.

### Description of the Related Art

Sterilization is used in a broad range of industrial and medical applications. Sterilization is the complete destruction or the irreversible inactivation of all the microorganisms in the sterilizer. Sterilization can be performed, for example, with heat or chemical treatment. Heat sterilization is normally done using steam. Some equipment cannot withstand the heat or the moisture of steam treatment. As a result, chemical sterilization is now commonly used.

Chemical disinfection or sterilization can be done using alcohols, aldehydes such as formaldehyde, phenols, ozone, ethylene oxide, chlorine dioxide, peracetic acid, or hydrogen peroxide. Hydrogen peroxide and ethylene oxide are commonly used for chemical sterilization.

U.S. Patent No. 4,643,876, discloses an exemplary sterilization process in which a vaporizable germicide such as hydrogen peroxide is introduced into an evacuated sterilization chamber. The vaporizable germicide vaporizes and is allowed to diffuse throughout the chamber and onto the items to be sterilized. After a period of time, electrical energy is applied to an electrode to form a plasma to complete the sterilization cycle.

The STERRAD® Sterilization System is an exemplary hydrogen peroxide sterilization system utilizing low-temperature plasma, commercially available from Advanced Sterilization Products, Irvine, California. The system employs an automated delivery system in which a measured amount of the liquid germicide, typically aqueous hydrogen peroxide, is delivered to the sterilization chamber. Measured portions of the liquid germicide are provided in rupturable cells within a liquid cassette housing. The cassette and the delivery system are fully described in the Williams et al. patents, U.S. Patent Nos. 4,817,800, issued April 4, 1989; 4,913,196, issued April 3, 1990; 4,938,262, issued July 3, 1990; and 4,941,518, issued July 17, 1990.

With the nature of the art, many and/or large pieces of equipment are required to be sterilized in a short period of time; therefore, large sterilization chambers are needed to accommodate such volumes. Although the cassette and the delivery system are adequate for most applications, the volumes of vaporizable germicide that can be delivered to the sterilization chamber with the cassette delivery system are limited to incremental volumes of single cells on the cassette. Various sizes of large sterilization chambers require an administration of large amounts of vaporizable germicide. Delivering large volumes of sterilant would require a large number of cassettes. There is a need for a single piece of apparatus to deliver vaporizable germicide to large chambers of different sizes all at one time. Additionally, with the administration of large volumes of liquid samples, the bulkiness of the large volume of liquid sample may add to the hazard of handling for the operator. Because of the inherent toxicity of certain germicides, there is a need for a delivery system to the sterilization chambers that is easy to handle.

U.S. Patent No. 4,744,955 discloses an adjustable volume pipette sampler in which the volume of delivered liquid is controlled by a pair of barrels that are axially aligned, retained together by complementary screw threads, and adjustable by relative rotation of the barrels.

While the adjustable volume pipette sampler offers a means to deliver varying volumes of liquid samples, the sampler is hard to manipulate, especially with large volumes of liquid samples. To adjust between large volumes requires a great deal of time and energy of the operator. Therefore, there is a need for a simple, safe, and quick system to deliver large amounts of liquid samples.

There is a need for a simple, safe, precise, and inexpensive system for introducing vaporizable germicide into a sterilization chamber in which the amount of vaporizable germicide can be varied. There is a need for a simple vaporizable germicide delivery system that can deliver a wide range of volumes, including very large volumes, of vaporizable germicide to match the needs of various sizes of sterilization chambers or rooms.

### Summary of the Invention

One aspect of the invention involves an adjustable accumulator for delivering a chemical, which can be a gas, liquid, or solid. The adjustable accumulator includes a hollow outer tube having a first open end and a second end connected to a first valve; and a hollow inner tube having a first open end and a second end connected to a second valve. The first open end of the inner tube is adapted to movably and securely connected to the first open end of the outer tube so as to retain the chemical within the volume, with the volume being the space enclosed within the outer tube, the inner tube, the first valve, and the second valve. Preferably, the outer tube and the inner tube are cylindrical. The adjustable accumulator can include at least one O-ring. To hold the inner tube and outer tube in a desired position, a retainer can be used. Examples of retainers include setscrews, spacers, and shafts or jacks.

In an embodiment of the invention, the adjustable accumulator has an outer tube which has at least one drum. The drum has an interior, such that the volume of retainment of the chemical includes the interior when the inner tube is adjusted to a position so that the interior of the drum is in fluid communication with the volume. The drum can be oval, circular, or any polygonal shape and does not necessarily surround the entire outer tube.

In another embodiment of the invention, the adjustable accumulator has an outer tube which has a drum that has at least one partition to separate the interior of the drum into at least two compartments. The compartment has a compartment interior where the volume of retainment of the chemical includes the compartment interior when the inner tube is adjusted to a position so that the compartment interior is in fluid communication with the volume. The compartment can be detachable from the adjustable accumulator. The compartments can be substantially identical in size and shape. The partition can be downwardly sloped toward the inner tube.

Another aspect of the invention involves a system for sterilizing equipment. The system includes an adjustable accumulator, a germicide reservoir to hold germicide, preferably hydrogen peroxide, and a sterilization chamber. The adjustable accumulator includes a hollow outer tube having a first open end and a second open end connected to a first valve; and a hollow inner tube having a first open end and a second end connected to a second valve. The germicide reservoir and the sterilization chamber are in fluid communication with the adjustable accumulator. The system can further include a vaporizer, vacuum pump, and/or source of plasma. The system can situate the first valve to be located between the adjustable accumulator and the sterilization chamber and the second valve to be located between the adjustable accumulator and the reservoir.

In other embodiments of the invention, the system can include the adjustable accumulator with the outer tube that has at least one drum. The drum can have at least one partition to separate the interior of the drum into at least two compartments.

Another aspect of the invention involves a method for sterilizing an article in a chamber. The method includes placing the article in the chamber; delivering a volume of germicide, preferably hydrogen peroxide, from a reservoir containing germicide to the chamber via the adjustable accumulator; and contacting the germicide with the article. The method can further include evacuating the chamber, vaporizing the germicide, opening and/or closing the second valve between the adjustable accumulator and the reservoir, opening and/or closing the first valve between the adjustable accumulator and the chamber, and/or moving the inner tube and/or outer tube so as to adjust the volume of the adjustable accumulator.

In other embodiments of the invention, the method can include the adjustable accumulator with the outer tube that has at least one drum. The drum can have at least one partition to separate the interior of the drum into at least two compartments.

### Brief Description of the Drawings

FIGURE 1 is a schematic drawing showing a sterilization system and a cross section of an adjustable accumulator according to an embodiment of the invention;

FIGURES 2A-C show adjustable accumulators comprising an upper tube and a lower tube;

FIGURES 3A-C show the relative positions of the upper tube and the lower tube of the adjustable accumulator of Figure 2;

FIGURE 4 is a schematic drawing showing an adjustable accumulator with an upper tube and a lower tube comprising a drum made up of a plurality of drum compartments that are separated by drum partitions, where a bottom edge of the upper tube is in contact with a bottom wall of the lower tube;

FIGURE 5 is a schematic drawing showing the adjustable accumulator of Figure 4, where a bottom edge of the upper tube is in contact with the first drum partition;

FIGURE 6 shows an embodiment of an adjustable accumulator in which retainers that hold an upper tube and a lower tube in place comprise setscrews;

FIGURE 7 shows an embodiment of an adjustable accumulator in which retainers that hold an upper tube and a lower tube in place comprise spacers;

FIGURE 8 shows an embodiment of an adjustable accumulator in which retainers that hold an upper tube and a lower tube in place comprise a shaft or jack;

FIGURE 9 shows a plurality of drums that extend from a lower tube and arc separated from each other;

FIGURE 10 shows a plurality of drum compartments that extend from a lower tube and are connected to each other;

FIGURE 11 shows a lower tube that consists of a drum with partitions;

FIGURE 12 shows a plurality of drum compartments that extend from a lower tube and are of different sizes;

FIGURE 13 is a schematic drawing of a top-view of an adjustable accumulator with a drum having a circular shape;

FIGURE 14 is a schematic drawing of a top-view of an adjustable accumulator with a drum having an oval shape;

FIGURE 15 is a schematic drawing of a top-view of an adjustable accumulator with a drum having a square shape;

FIGURE 16 is a schematic drawing of a top-view of an adjustable accumulator with a drum having a polygonal shape;

FIGURE 17 shows an embodiment of a drum of an adjustable accumulator, where the contact point between adjacent detachable drum compartments is a bottom wall of a drum compartment and a top wall of the adjacent drum compartment;

FIGURE 18 shows an embodiment of a drum of an adjustable accumulator, wherein the contact point between detachable adjacent drum compartments is a side wall of a drum compartment and a bottom wall of an adjacent drum compartment;

FIGURE 19 shows an embodiment of a drum of an adjustable accumulator, wherein the contact point between adjacent detachable drum compartments is a side wall of a drum compartment and a top wall of an adjacent drum compartment;

FIGURE 20 shows an arrangement of an O-ring to form a seal between an upper tube and a lower tube of an adjustable accumulator in which the O-ring is located on the inside of the outer tube;

FIGURE 21 shows an arrangement of an O-ring to form a seal between an upper tube and a lower tube of an adjustable accumulator in which the O-ring is located on the outside of the inner tube;

FIGURE 22 shows an arrangement of O-rings to form a seal between an upper tube and a lower tube of an adjustable accumulator in which the O-rings are located on the inside of the outer tube and on the outside of the inner tube;

FIGURE 23 shows an embodiment of a drum of an adjustable accumulator, wherein a drum partition includes an O-ring at the contact point with the upper tube;

FIGURE 24 shows an embodiment of an adjustable accumulator mounted with a reservoir and a vaporizer in which the transfer of vaporizable germicide between the components proceeds via gravity, pressure, or vacuum;

FIGURE 25 shows an embodiment of an adjustable accumulator in connection with a reservoir and a vaporizer via tubes in which the transfer of vaporizable germicide between the components proceeds via a vacuum;

FIGURE 26 is a schematic drawing of the sterilization system and the adjustable accumulator after a vaporizable germicide has been admitted into the upper tube of the adjustable accumulator;

FIGURE 27 is a schematic drawing of the sterilization system and the adjustable accumulator after a bottom edge of the upper tube has been raised and placing one drum compartment in fluid communication with the upper tube, allowing a vaporizable germicide in the upper tube to enter one drum compartment; and

FIGURE 28 is a schematic drawing of the sterilization system and the adjustable accumulator after a bottom edge of the upper tube has been raised and placing two drum compartments in fluid communication with the upper tube, allowing a vaporizable germicide in the upper tube to enter the two drum compartments.

| Reference Numerals in Drawings | | | |
|---|---|---|---|
| 10 | sterilization chamber | 100 | top edge |
| 20 | adjustable accumulator | 110 | side wall |
| 24 | reservoir | 120 | bottom wall |
| 26 | vaporizable germicide | 130 | drum |
| 28 | vaporizer | 140 | drum bottom |
| 30 | first valve | 150 | drum side |
| 32 | second valve | 160 | drum top |
| 36 | vacuum pump | 170 | drum partition |
| 40 | valve | 180 | drum compartment |
| 50 | upper tube | 190 | setscrew |
| 60 | lower tube | 200 | notch |
| 70 | bottom edge | 210 | spacer |
| 80 | side wall | 220 | shaft or jack |
| 90 | top wall | 230 | O-ring |

### Detailed Description of the Preferred Embodiment

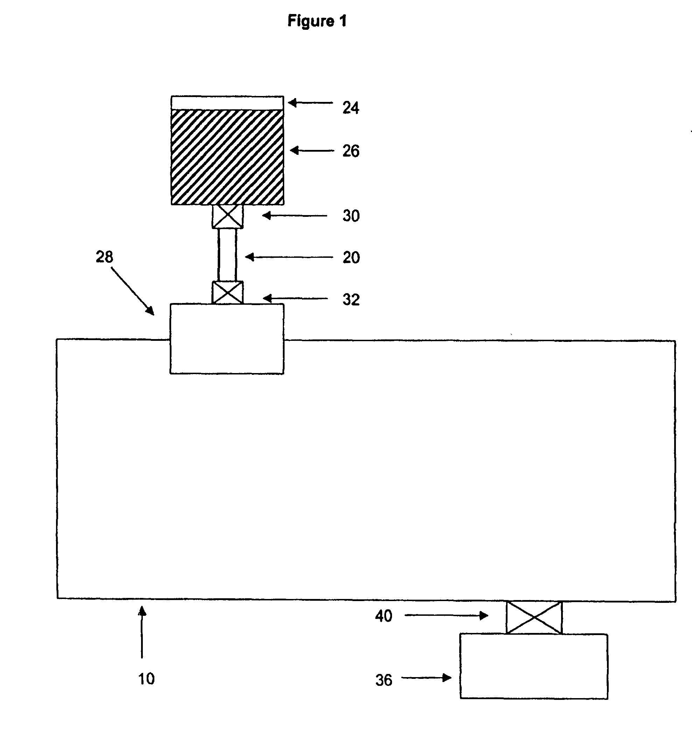

Figure 1 is a schematic diagram of a sterilization chamber 10 with an adjustable accumulator 20 according to one embodiment of the invention. The sterilization chamber 10 and its components and methods of use are described in detail in U.S. Patent No. 4,756,882, issued July 12, 1988; U.S. Patent No. 5,656,238, issued August 12, 1997; and U.S. Patent No. 6,060,019, issued May 9, 2000. The adjustable accumulator 20 is mounted between a reservoir 24 that contains a vaporizable germicide 26, and a vaporizer 28, which is located above and is in fluid communication with the sterilization chamber 10. The term "germicide" as used herein includes both sterilants and disinfectants. First and second valves 30 and 32 are located between the reservoir 24 and the adjustable accumulator 20 and between the adjustable accumulator 20 and the vaporizer 28. A vacuum pump 36 and a valve 40 are fluidly connected with the sterilization chamber 10.

The accumulator can be designed to be adjustable to have the capability to deliver different volumes of materials. Although the adjustable accumulator 20 is described in the context of an adjustable accumulator for delivering vaporizable germicide 26 to a sterilization chamber or room 10, it is to be understood that the application of the adjustable accumulator 20 to sterilization is illustrative only. The adjustable accumulator 20 of the present invention has many uses, and the example of delivering vaporizable germicide to a sterilization chamber 10 is exemplary and is not meant to be limiting. Further, the adjustable accumulator 20 can be used to deliver gases, liquids, solids, and slurries of solids in one or more liquids.

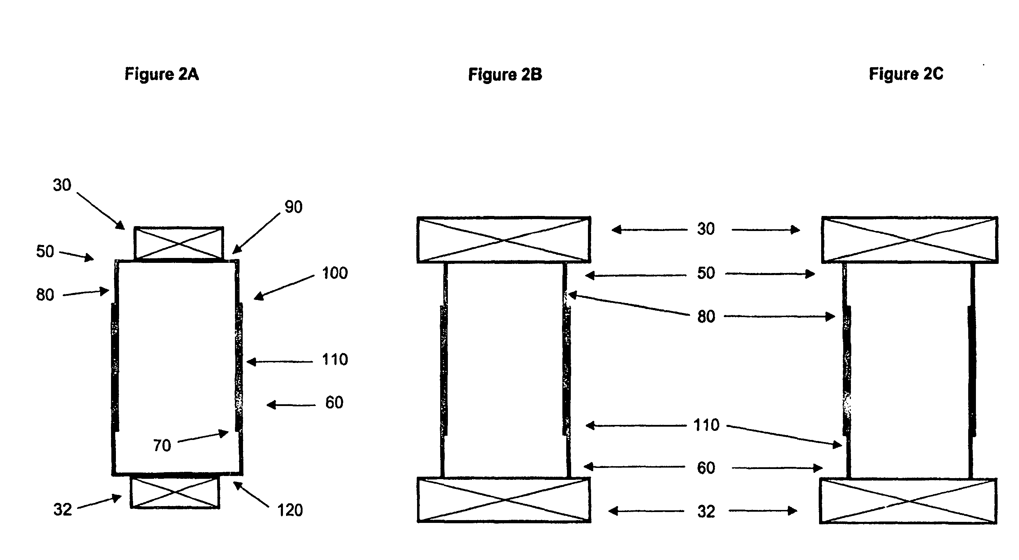

The invention will now be described with reference to Figures 2-3, which illustrate basic embodiments of the adjustable accumulator. Figures 2A-C are schematic diagrams of the adjustable accumulator, which comprises two parts: an upper tube 50 and a lower tube 60. The upper tube 50 comprises a bottom edge 70, a side wall 80, and a top wall 90 that is connected to the first valve 30. The lower tube 60 comprises a top edge 100, a side wall 110, and a bottom wall 120 that is connected to the second valve 32. The lengths of longitudinal axes of the upper tube 50 and the lower tube 60 can be of any length and are not necessarily equal to each other. One tube fits into the other tube to be sealingly engaged and to create a chamber in the interior. In the preferred embodiment of the invention, the upper tube 50 is the inner tube and the lower tube 60 is the outer tube that surrounds the upper tube 50 and is sealingly engaged with the upper tube 50. In another embodiment, the lower tube 60 is the inner tube and the upper tube 50 is the outer tube that surrounds the lower tube 60 and is sealingly engaged with the lower tube 60. In both instances, the inner tube is movable within the outer tube so that the inner tube can be placed in different positions relative to the outer tube; therefore, the volume of the chamber is adjustable. The tubes can take any shape, preferably circular, oval, or any polygonal shape, as long as the inner tube is sealingly engaged with the outer tube.

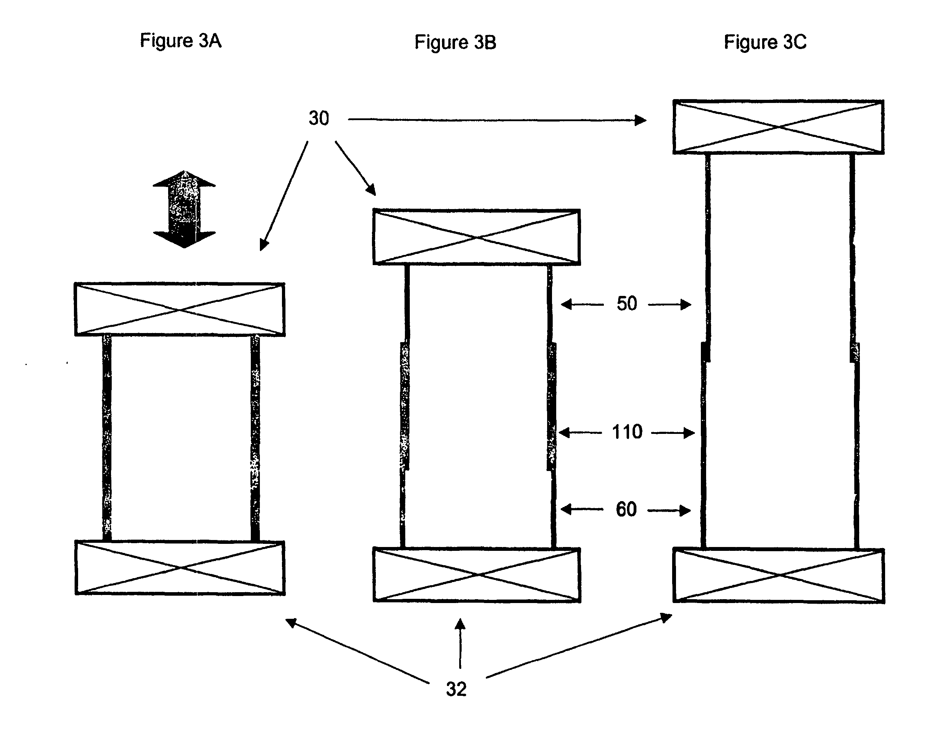

Figures 3A-C show the relative positions of the upper tube 50 and the lower tube 60 of the preferred embodiment in which the upper tube 50 is the inner tube and the lower tube 60 is the outer tube. Although Figures 3A-C describe the dynamic movement of one embodiment of the adjustable accumulator, the example is not meant to be limiting. For example, the description of the dynamic movement of the invention applies also to the situation where the upper tube 50 is the outer tube and the lower tube 60 is the inner tube. The adjustable accumulator is shown in the lowest position in Figure 3A. In Figure 3A, the length of the longitudinal axis of the chamber that is created by the upper tube 50 and the lower tube 60 is equal to about the length of the upper tube 50 or lower tube 60, whichever is longer. In Figure 3B, the upper tube 50 and lower tube 60 have been shifted from the lowest position in Figure 3A to alter the volume of the chamber. The upper tube 50 and lower tube 60 can hold any intermediate position where the side wall 80 of the upper tube 50 can overlap with the side wall 110 of the lower tube 60. As shown in Figure 3C, the maximum length of the longitudinal axis of the chamber is equal to about the sum of the lengths of the longitudinal axes of the upper tube 50 and the lower tube 60.

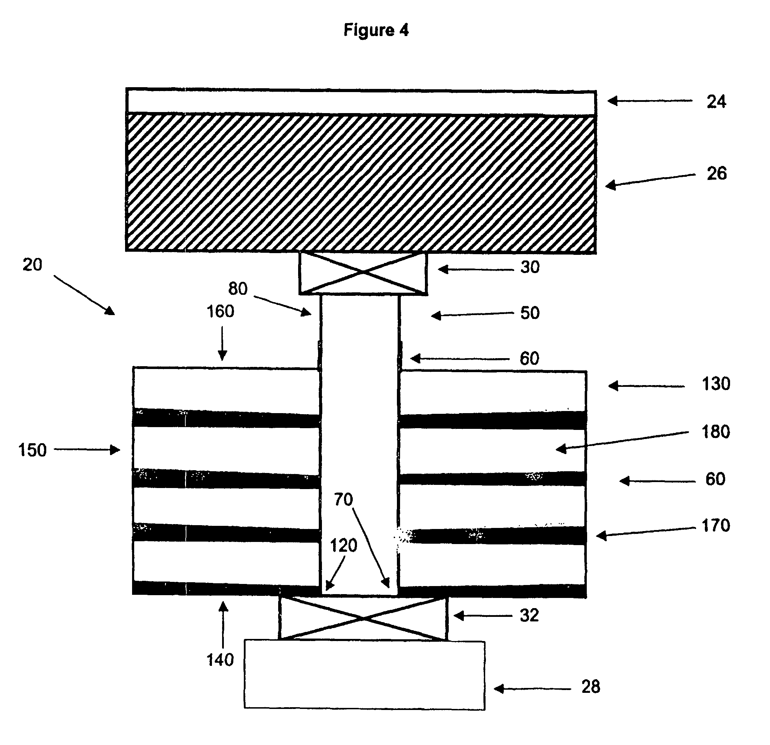

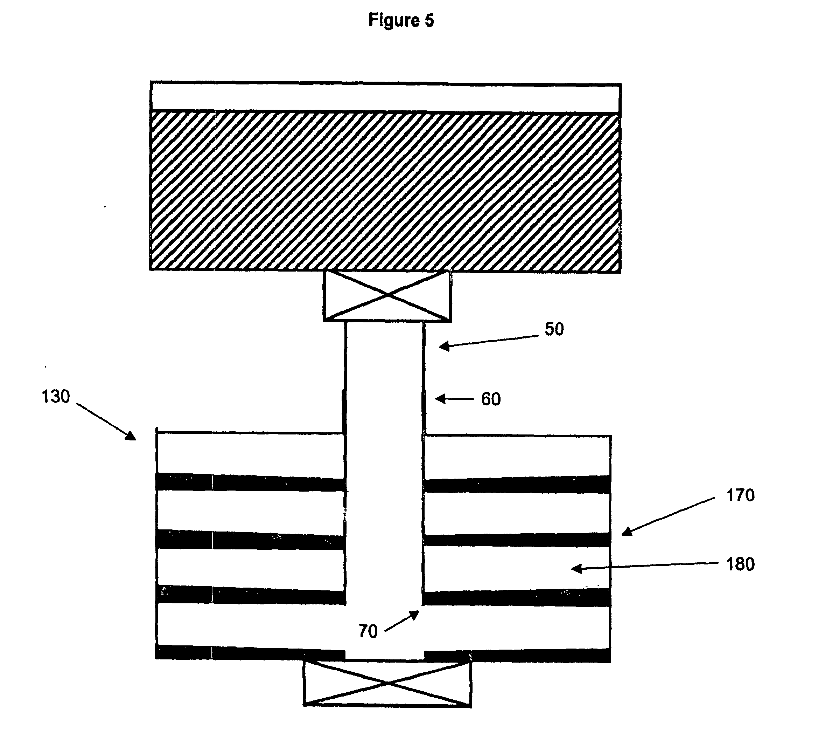

The invention will now be described with reference to Figures 4 and 5, which illustrate other embodiments of the invention. The adjustable accumulator comprises compartments to accommodate additional volume. Figure 4 is a schematic diagram of the adjustable accumulator 20 situated between the reservoir 24 that contains vaporizable germicide 26 and the vaporizer 28. The adjustable accumulator 20 of Figure 4 and 5 comprises the upper tube 50 and the lower tube 60, where the upper tube 50 fits in the lower tube 60 and is sealingly engaged with the lower tube 60. The lower tube 60 comprises a drum 130. The drum 130 may be situated anywhere along the longitudinal axis of the lower tube 60. Alternatively, the lower tube 60 consists of the drum 130. The drum 130 needs not necessarily to surround the entire upper tube 50. The drum comprises a drum bottom 140, a drum side 150, and a drum top 160. Situated in the interior of the lower tube 60, the upper tube 50 is preferably a vertically movable cylindrical tube whose length is equal to or exceeds the height of the lower tube 60 and has the ability to traverse the center of the lower tube 60. A plurality of drum partitions 170 that extend from the drum side 150 are able to make contact with the vertically movable upper tube 50, depending on the position of the upper tube 50. The drum 130 is divided into a plurality of drum compartments 180 by the plurality of drum partitions 170. The adjustable accumulator 20 is in fluid communication with the reservoir 24 and with the vaporizer 28 through gas-tight seals. The upper tube 50 communicates with the reservoir 24 through the first valve 30, located between the upper tube 50 and the reservoir 24. The lower tube 60 communicates with the vaporizer 28 through the second valve 32, located between the lower tube 60 and the vaporizer 28.

In Figure 4, the upper tube 50 is positioned at the lowest position with the bottom edge 70 of the upper tube 50 in contact with the bottom wall 120 of the lower tube 60. The upper tube 50 traverses the lower tube 60. Alternatively, the lowest position of the adjustable accumulator 20 could position the bottom edge 70 of the upper tube 50 in contact with the drum bottom 140. When the upper tube 50 is in the lowest position, all drum partitions 170 fit tightly against the upper tube 50, forming a gas-tight seal with the upper tube 50. Because the bottom edge 70 of the upper tube 50 is in contact with the bottom wall 120 of the lower tube 60 or the drum bottom 140, none of the drum compartments 180 is in fluid communication with the interior of the upper tube 50.

In Figure 5, the upper tube 50 has been raised such that the bottom edge 70 of the upper tube 50 makes contact and is sealingly engaged with the first drum partition 170, thereby placing the lowest drum compartment 180 into fluid communication with the interior of the upper tube 50. The interior of any particular drum compartment 180 is blocked from the upper tube 50 when the bottom edge 70 of the upper tube 50 is in contact with a drum partition 170 at a level lower than the particular drum compartment 180. The interior of any particular drum compartment 180 is exposed to the interior of the upper tube 50 when the bottom edge 70 of the upper tube 50 is in contact with a drum partition 170 at a level higher than the particular drum compartment 180. Moving the upper tube 50 up and down varies the number of drum partitions 170 that are in contact with the upper tube 50 and, therefore, the number of drum compartments that are in fluid communication with the interior of the upper tube 50. Under certain embodiments, the fluid communication between the drum compartment 180 and the interior of the upper tube 50 can also be achieved by resting the bottom edge 70 of the upper tube 50 in between two drum partitions 170.

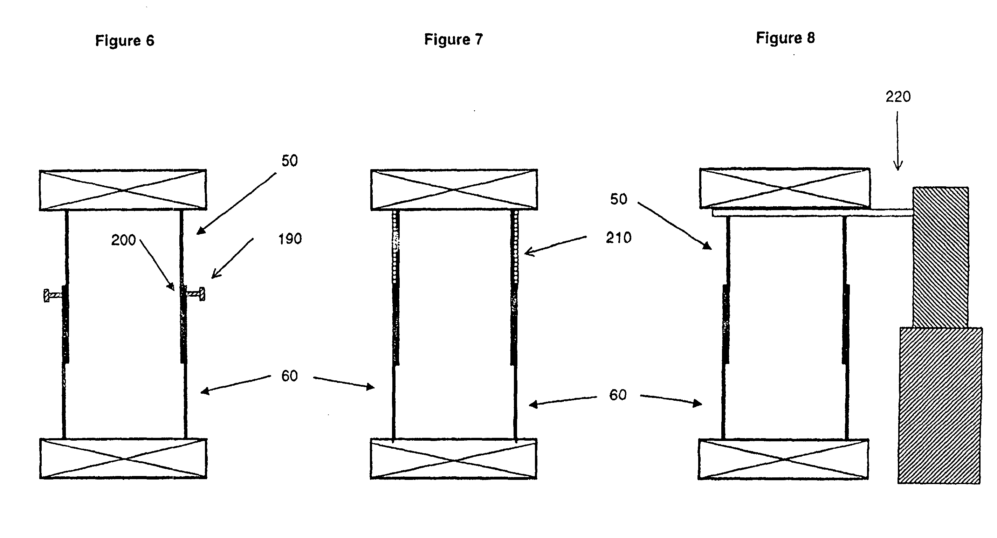

The vertically movable upper tube 50 and lower tube 60 can be held at various vertical levels by retainers. Figures 6-8 show embodiments displaying mechanisms to secure the upper tube 50 and lower tube 60 in place in which the upper tube 50 is the inner tube and the lower tube 60 is the outer tube, though the same mechanisms can work for other embodiments of the invention as well. Figure 6 shows an embodiment in which a plurality of setscrews 190 fit through the lower tube 60 into a plurality of notches 200 of the upper tube 50 to hold the lower tube 60 and the upper tube 50 in place. The plurality of notches 200 are sized so as to fit with the plurality of setscrews 190. The notches 200 are located along different lengths of the longitudinal axis of the lower tube 60 on its outer diameter and do not extend to the inner diameter of the lower tube 60.

In Figure 7, the retainer is a spacer 210 that fits exactly into a space along the side wall of the inner tube, whether the upper tube 50 or lower tube 60, that is exposed when the inner tube and outer tube are positioned in an intermediate position. The spacer 210 is placed between the edge of the outer tube and end of the inner tube next to the valve to prevent motion of the inner tube or outer tube. The spacer 210 can be of any length to accommodate various intermediate positions of the inner tube and the outer tube.

In Figure 8, the retainer is a shaft or jack 220 with a side arm used to support the upper tube 50 by directly connecting to the upper tube 50. The shaft or jack 220 sits in a position to be able to hold the upper tube 50 in place. The shaft or jack 220 can be operated manually, hydraulically, pneumatically, mechanically, or electrically with a motor, such as a stepper motor. The apparatus is not limited by the use of the mechanisms mentioned as retainers; any device that can hold the upper tube 50 in place at various vertical levels is considered to be a suitable retainer.

Referring again to Figure 1, the reservoir 24 and the adjustable accumulator 20 are in fluid communication when the first valve 30 is opened. Closing the first valve 30 prevents the vaporizable germicide 26 from entering the adjustable accumulator 20. The junction of the reservoir 24 and the adjustable accumulator 20 is preferably able to hold a gas-tight seal. The first valve 30 may comprise two pieces in which one piece may be directly affixed to the top of the upper tube 50 or to the bottom of the reservoir 24. Then, the adjoining piece of the first valve 30 on the opposing end preferably fits with the first portion of the first valve 30 as to create a gas-tight seal. The gas-tight seals will be discussed in detail below.

Referring again to Figure 1, opening the second valve 32 allows fluid communication between the adjustable accumulator 20 and the vaporizer 28. Closing the second valve 32 prevents the vaporizable germicide 26 from entering the vaporizer 28. The junction of the adjustable accumulator 20 and the vaporizer 28 is preferably gas-tight. Specifically, the second valve 32 is located at the bottom of the lower tube 60. Like the first valve 30, the second valve 32 may comprise two pieces in which one piece may be directly affixed to the bottom of the lower tube 60 or to the top of the vaporizer 28. Then, the adjoining piece of the second valve 32 on the opposing end preferably fits with the first portion of the second valve 32 as to create a gas-tight seal. The gas-tight seals will be discussed in detail below.

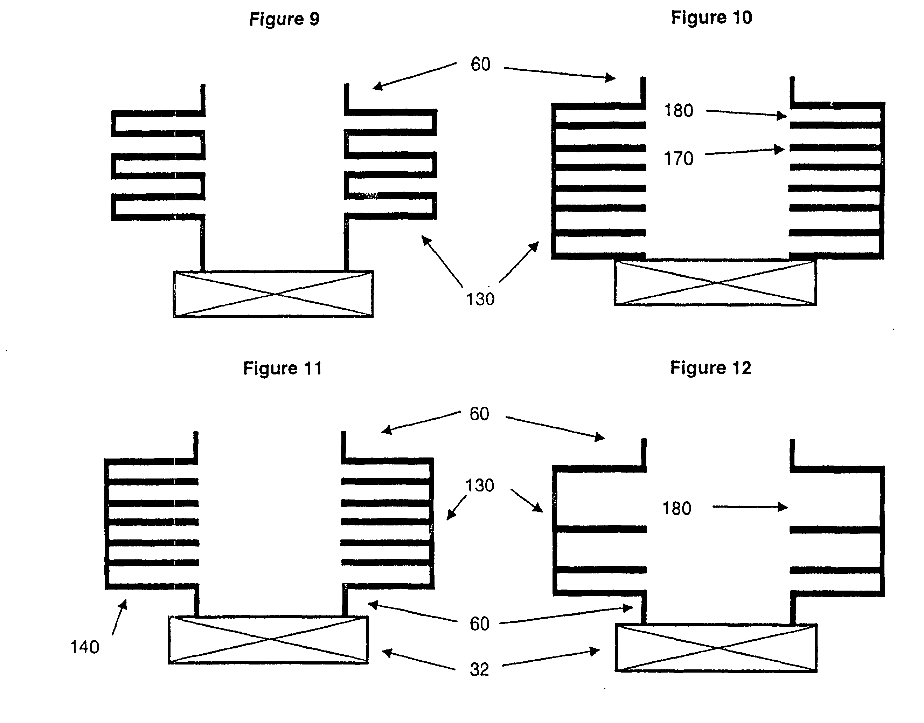

The drum 130 can extend from the lower tube 60 in several ways. Figures 9-12 show the lower tube 60 in several embodiments. Figure 9 shows the plurality of drums 130 extending from the lower tube 60 where the drums 130 are separated from each other. Figure 10 shows the drum 130 extending from the lower tube 60 where the drum compartments 180 are connected to each other and separated with drum partitions 170. Figure 11 shows the plurality of drum compartments 180 extending from the lower tube 60 where the drum bottom 140 does not rest on the second valve 32. Figure 12 shows the plurality of drum compartments 180 extending from the lower tube 60 where the drum compartments 180 are of different sizes.

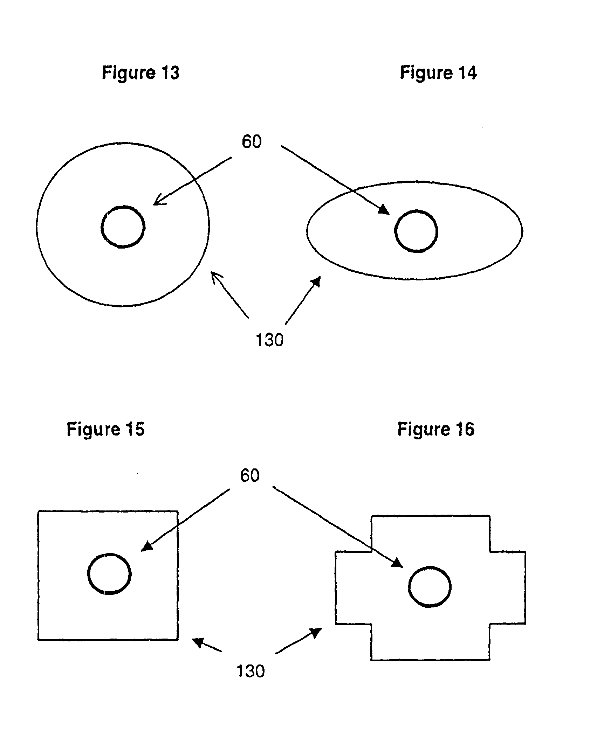

The upper tube 50, the lower tube 60, and the drum 130 can have any shape so as long as the upper tube 50 fits into and is movable within the lower tube 60. In exemplary embodiments, the shape of the lower tube 60 or drum 130 is circular, oval, square, or twelve-sided polygonal, as seen in Figures 13-16. For certain embodiments, the drum 130 may not surround the entire lower tube 60. The lower tube 60 and the drum 130 may vary in size so as to allow delivery of a wide range of volumes of the vaporizable germicide to match the needs of various sizes of sterilization chambers. In embodiments appropriate for sterilizers, the drum may have a volume up to about 100 liters, preferably about 1 to about 1000 milliliters, more preferable about 2 to about 100 milliliters.

There is no limit to the total number of drum compartments 180 that occupy the drum 130. In one embodiment, the drum compartments 180 are detachable from the adjustable accumulator 20. The horizontally extending drum compartments 180 are stacked one above another. In one embodiment, the drum compartments 180 detach one at a time from the adjustable accumulator 20. In another embodiment, more than one drum compartment 180 are fused together to from a single unit and are detached from the adjustable accumulator 20 as a unit.

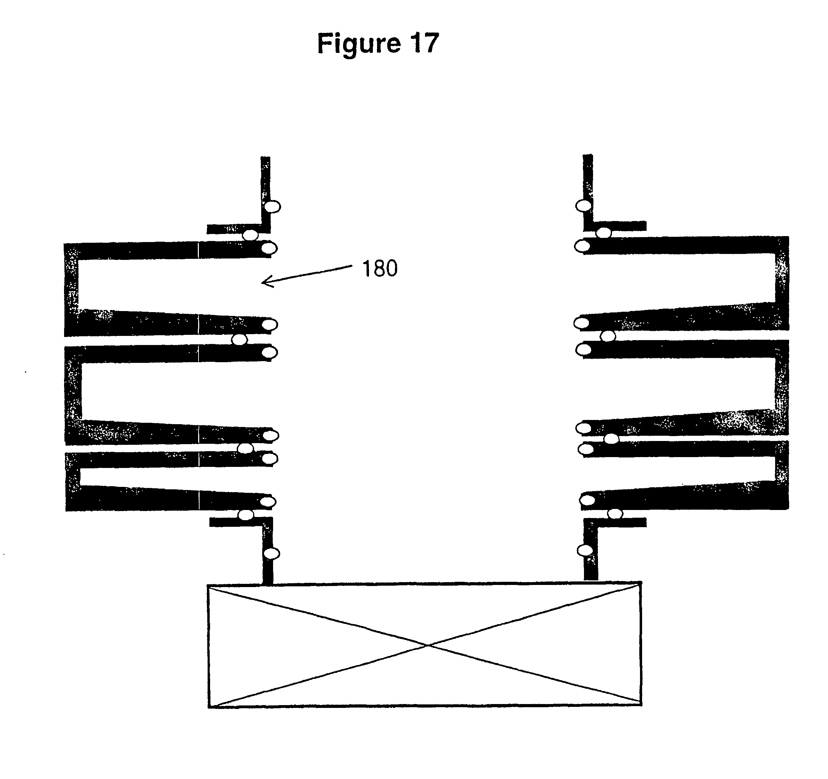

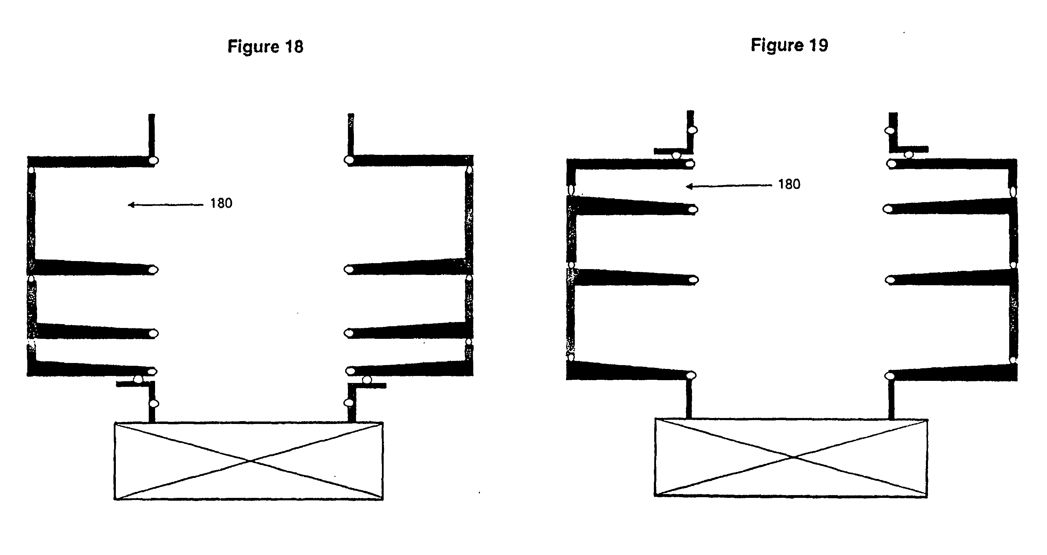

When the drum compartments 180 stack one on top of another and are detachable from one another, the contact points between the adjacent detachable drum compartments 180 may be oriented differently in different embodiments. Also, shown in Figures 17-19, the drum partitions 170 can be downwardly sloped to allow thorough delivery of germicide. Figure 17 shows an embodiment in which the contact point between the adjacent detachable drum compartments 180 is a bottom wall of a drum compartment and a top wall of the adjacent drum compartment. Figure 18 shows an embodiment in which the contact point between the adjacent detachable drum compartments 180 is a side wall of a drum compartment and a bottom wall of the adjacent drum compartment. Figure 19 shows an embodiment in which the contact point between the adjacent detachable drum compartments 180 is a side wall of a drum compartment and a top wall of the adjacent drum compartment.

The adjustable accumulator 20 can be made from a wide range of materials, including metal, glass, or plastic. Suitable metals include steel or aluminum. Aluminum and TEFLON™ are exemplary materials for forming the adjustable accumulator 20. TEFLON™ is a tradename for polytetrafluoroethylene.

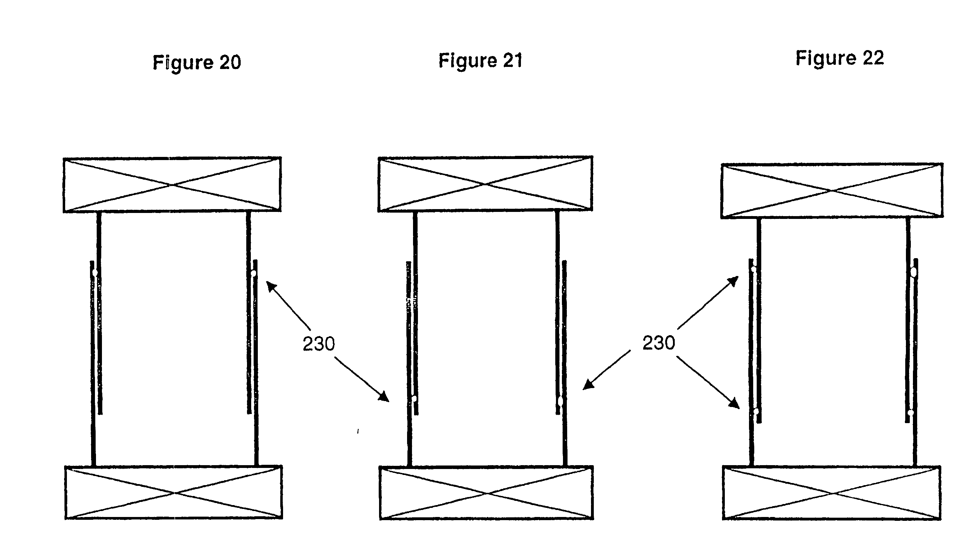

Preferably, the apparatus of the invention includes gas-tight seals in the adjustable accumulator 20 between the upper tube 50 and the lower tube 60. A seal between the bottom edge 70 of the upper tube 50 and the top edge 100 of the lower tube 60 can be achieved in several ways, depending on the material from which the adjustable accumulator 20 is fabricated. In one embodiment, both the upper tube 50 and the lower tube 60 are made of TEFLON™. In another embodiment, one of the tubes is made of TEFLON™ and the other tube is made of metal. In another embodiment, one of the tubes is made of TEFLON™ and the other tube is made of glass. If the upper tube 50 and the lower tube 60 are properly fabricated, the contact between the upper tube 50 and the lower tube forms a seal. In another embodiment, both the upper tube 50 and the lower tube 60 are made of metal. As shown in Figures 20-22, O-rings 230 or packing can be placed on the upper tube 50 and/or the lower tube 60 in different arrangements to form a seal. The O-rings 230 can be placed on the inside of the outer tube, as shown in Figure 20, or on the outside of the inner tube, as shown in Figure 21, or on both the inside of the outer tube and the outside of the inner tube, as shown in Figure 22.

If O-rings 230 or packing are used in the adjustable accumulator 20, the O-rings 230 or packing are preferably formed of a material that is resistant to the vaporizable germicide 26 that is used. VITON™ is an exemplary material for forming the O-rings 230 or packing. TEFLON™ or silicone may also be used to form the O-rings 230 or packing.

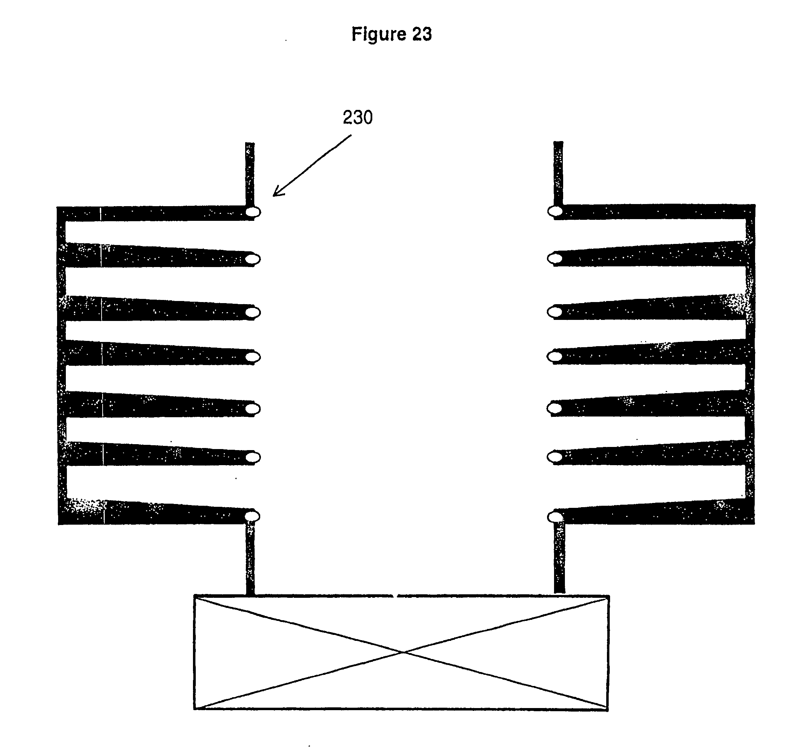

Likewise, in the case of the adjustable accumulator 20 of Figure 4 which has the drum 130, the apparatus of the invention includes gas-tight seals in the adjustable accumulator 20 between the side wall 80 of the upper tube 50 and the drum partition 170. If the upper tube 50 is positioned in the lowest position as shown in Figure 4, then the bottom edge 70 of the upper tube 50 is aligned with the lowest drum partition 170 or the bottom wall 120 of the lower tube 60. A seal between the bottom edge 70 of the upper tube 50 and the drum partition 170 can be achieved in the same manner as that of the seals between the upper tube 50 and the lower tube 60 mentioned previously. If O-rings 230 are to be used, Figure 23 shows one possible arrangement of O-rings 230 to accommodate the sealing of the upper tube 50 with the drum partitions 170.

The apparatus of the invention may require gas-tight seals in the adjustable accumulator 20 at the contact points between the adjacent detachable drum compartments 180. A seal between the adjacent detachable drum compartments 180 of the adjustable accumulator 20 can be achieved in several ways, depending on the material from which the adjustable accumulator 20 is fabricated. If the adjustable accumulator 20 is made of TEFLON™, the walls can be fabricated so that the contact between the TEFLON™ walls of adjacent detachable drum compartments forms a seal. If the adjustable accumulator 20 is made of metal, glass, or plastic, O-rings 230 or packing are generally needed to make a proper seal between the walls of adjacent detachable drum compartments 180. If O-rings 230 are to be used, Figures 17-19 show possible arrangements of O-rings 230 to accommodate the sealing of the drum compartments 180.

Preferably, the apparatus of the invention includes gas-tight seals at the junction between the first valve 30 and the reservoir 24, and between the second valve 32 and the sterilizer 10. Examples of gas-tight seals are the same as those mentioned previously for seals between walls of adjacent detachable drum compartments 180. Alternatively, if the valves are made out of glass, the portions of the valves can be made as a ground-glass joint to provide a gas-tight seal.

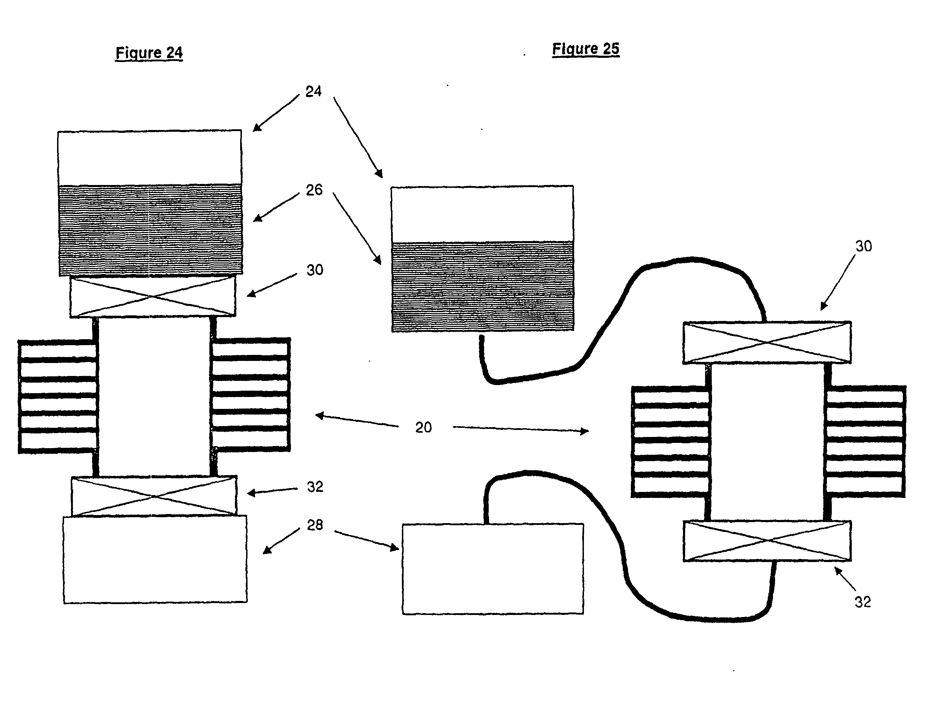

Figures 24-25 show different embodiments with regards to the accumulation of vaporizable germicide 26 in the adjustable accumulator 20. Figure 24 shows the adjustable accumulator 20 that is connected below the reservoir 24 and above the vaporizer 28. The first valve 30 is opened to transfer the vaporizable germicide 26 from the reservoir 24 to the adjustable accumulator 20. Likewise, after the vaporizable germicide 26 has collected in the adjustable accumulator 20, the second valve 32 is opened to transfer the vaporizable germicide 26 from the adjustable accumulator 20 to the vaporizer 28. Both transfers of vaporizable germicide can be conducted with either gravity, pressure, or vacuum. In another embodiment shown in Figure 25, tubes connect the adjustable accumulator 20 to the reservoir 24 and the vaporizer 28. The transfer of vaporizable germicide proceeds because of vacuum. With the second valve 32 opened and the first valve 30 closed, the vacuum pump evacuates the adjustable accumulator 20 and the vaporizer 28. Then, with the second valve 32 closed and the first valve 30 opened, the adjustable accumulator 20 is filled with vaporizable germicide 26 from the reservoir 24 via the vacuum that had been created. Finally, with the first valve 30 closed and the second valve 32 opened, the vaporizable germicide 26 is transferred from the adjustable accumulator 20 to the vaporizer 28.

Returning again to Figure 1, the accumulation of vaporizable germicide in the adjustable accumulator will now be discussed by applying an embodiment that uses either the gravity-fill, pressure-fill, or vacuum-fill method; however, the adjustable accumulator comprises other embodiments and this example is not viewed as limiting. The vaporizable germicide 26 is placed in the reservoir 24 above the first valve 30. The vaporizable germicide 26 can be any liquid vaporizable germicide including aqueous hydrogen peroxide, peracetic acid, chlorine dioxide, ozone, or formaldehyde. In a preferred embodiment, the vaporizable germicide 26 comprises aqueous hydrogen peroxide. In another preferred embodiment, the vaporizable germicide 26 is approximately 20-60 wt% aqueous hydrogen peroxide.

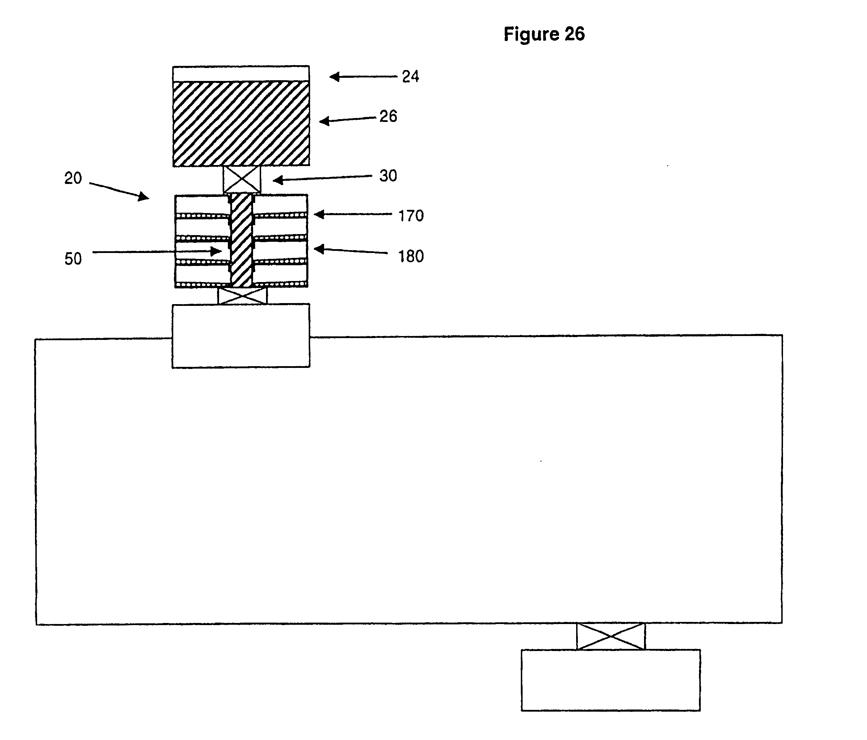

As shown in Figure 26, the first valve 30 between the reservoir 24 and the adjustable accumulator 20 is opened, allowing the vaporizable germicide 26 to enter the upper tube 50 of the adjustable accumulator 20. The upper tube 50 is positioned at the lowest position. All drum partitions 170 fit tightly against the upper tube 50 and none of the drum compartments 180 is in fluid communication with the interior of the upper tube 50. As a result, the vaporizable germicide 26 flows into the upper tube 50 alone.

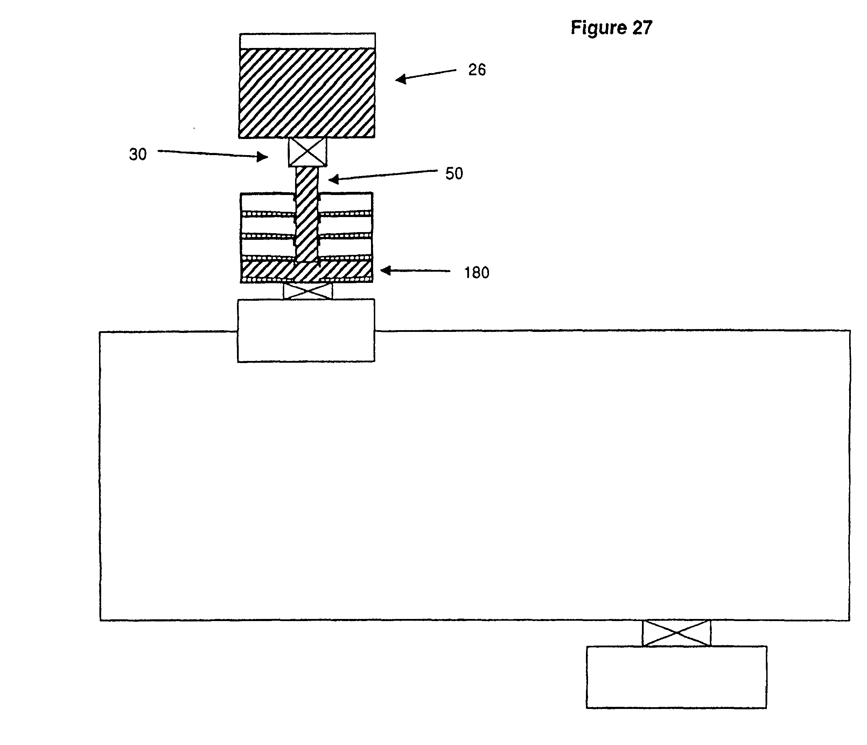

In Figure 27, the upper tube 50 has been raised to expose the lowest drum compartment 180 into fluid communication with the interior of the upper tube 50, allowing vaporizable germicide 26 to enter the lowest drum compartment 180 upon opening the first valve 30. The drum compartment 180 provides additional volume to the upper tube 50 and allows delivery of a larger volume of the vaporizable germicide 26 than the upper tube 50 alone. The upper tube 50 can be held in place by the methods described previously.

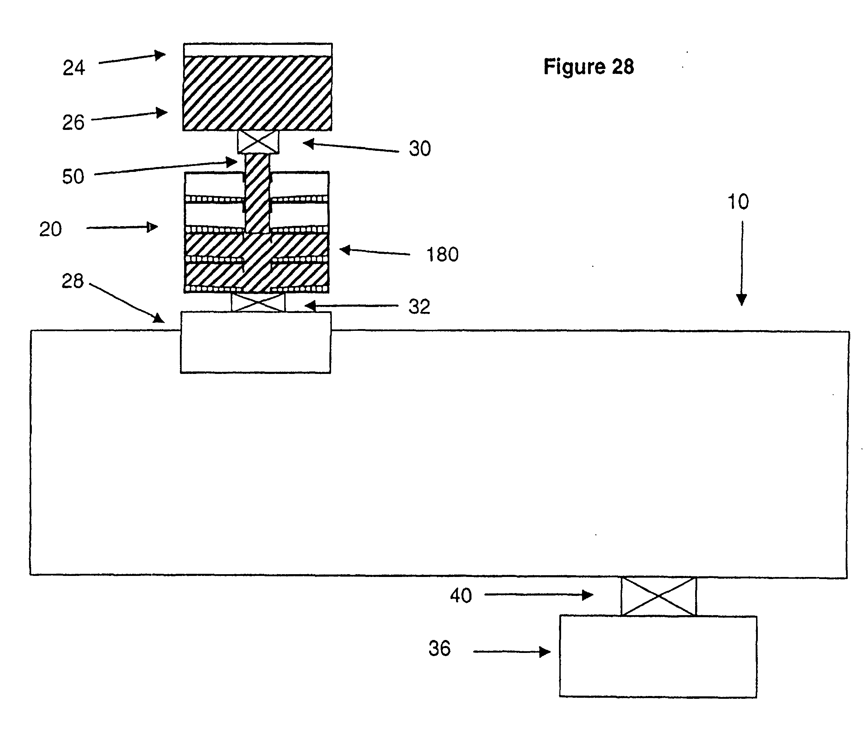

In Figure 28, the upper tube 50 has been raised to expose the two lowest drum compartments 180 and allowing vaporizable germicide 26 to enter the two lowest drum compartment 180 upon opening the first valve 30. Raising the upper tube 50 further places additional drum compartments into fluid communication with the interior of the upper tube 50. Additional compartments provide even more volume of vaporizable germicide.

The volume of germicide contained in the adjustable accumulator can be varied by adjusting the level of the upper tube 50. The drum compartments 180 offer additional volume to the upper tube 50, when the drum compartments 180 are exposed to the interior of the upper tube 50. The drum compartments 180 allow delivery of a larger volume of germicide than the upper tube 50 alone. If the upper tube 50 is moved to a higher level, more drum compartments 180 are in fluid communication with the interior of the upper tube 50, thereby increasing the overall volume of vaporizable germicide available for delivery into the sterilization chamber. The adjustable accumulator allows for the delivery of a wide range of large volumes of sterilant to match the needs of various sizes of sterilization chambers. Consequently, the adjustable accumulator offers a simple, safe, and quick system to deliver sterilant to sterilization chambers.

After the desired amount of the vaporizable germicide 26 has accumulated in the adjustable accumulator 20, and the pressure of the sterilization chamber 10 reaches the desired pressure, the second valve 32 is then opened to transfer the vaporizable germicide 26 to the vaporizer 28. The vaporizer 28 is fluidly connected to the interior of the sterilization chamber 10. The vaporizer is preferably maintained at a temperature of approximately 50 to approximately 70o C. As the vaporizable germicide 26 enters the heated vaporizer 28, the vaporizable germicide 26 vaporizes, and the vapor enters the sterilization chamber 10. The germicide vapor contacts the equipment to be sterilized (not shown) in the sterilization chamber 10, sterilizing the equipment. Optionally, plasma is introduced into or is generated in the sterilization chamber 10 to enhance the sterilization by the germicide vapor or to remove residual germicide.

When the vaporizable germicide 26 comprises hydrogen peroxide and water, accumulating larger volumes of vaporizable germicide 26 in the adjustable accumulator 20 has advantages over allowing smaller amounts of vaporizable germicide 26 to enter the vaporizer 28. Water has a higher vapor pressure than hydrogen peroxide. If the valve 40 between the sterilization chamber 10 and the vacuum pump 36 is left open when the vaporizable germicide 26 is vaporizing in the vaporizer 28, water is preferentially removed from the sterilization chamber 10, because water has a higher vapor pressure than hydrogen peroxide, and the vapor in the sterilization chamber 10 is enriched in water vapor compared to the vaporizable germicide 26 in the vaporizer 28. Removing water from the aqueous hydrogen peroxide left in the vaporizer 28 by removing the water vapor in the sterilization chamber 10 concentrates the hydrogen peroxide in the vaporizer 28.

After a predetermined period of time, the valve 40 leading to the vacuum pump 36 is closed, and the concentrated hydrogen peroxide is allowed to vaporize from the vaporizer 28 into the sterilization chamber 10. The concentrated hydrogen peroxide in the vaporizer 28 vaporizes to produce a vapor which has a higher concentration of hydrogen peroxide than if water had not been removed from the aqueous hydrogen peroxide in the vaporizer 28 by preferential vaporization. The concentrated hydrogen peroxide vapor is more effective at sterilization than hydrogen peroxide vapor produced from a less concentrated solution of aqueous hydrogen peroxide.

With the use of the adjustable accumulator, the sterilization process is more effective and efficient. The adjustable accumulator provides a simple, safe, and precise system for introducing vaporizable germicide into a sterilization chamber in which the amount of vaporizable germicide can be varied. The adjustable accumulator can deliver a wide range of volumes of vaporizable germicide to match the needs of various sizes of sterilization chambers. When large amounts of hydrogen peroxide as the vaporizable germicide is administered to the sterilization chamber, water can be preferentially removed from the system under vacuum; this scenario portrays a more effective sterilization process because the vapor in the sterilization chamber is enriched with hydrogen peroxide.

## Claims

1. An adjustable accumulator (20) having a volume for delivering a chemical, said adjustable accumulator comprising:
a hollow inner tube (50) having a first open end (70) and a second end ( 90) connected to a first valve (30); wherein
a hollow outer tube (60) having a first open end (100) and a second end (120) connected to a second valve (32); and
said first open end (70) of the inner tube (50) is adapted to fit into and seal with the first open end (100) of the outer tube (60), thereby creating a chamber bounded by the outer tube (60), the inner tube (50), the first valve (30) and the second valve (32), to retain said chemical; wherein
said volume is the volume of space enclosed by said chamber, and wherein
the inner tube (50) is movable within the outer tube (60) to adjust said volume of said chamber.

2. The adjustable accumulator of Claim 1, wherein the outer tube (60) and the inner tube (50) are cylindrical.

3. The adjustable accumulator of Claim 1, wherein
the outer tube (60) further comprises at least one drum (130) extending from said outer tube (60); wherein
said drum (130) has an interior; and wherein
said volume comprises said interior when said inner tube (50) is adjusted to a position so that the interior of said drum (130) is in fluid communication with said volume.

4. The adjustable accumulator of Claim 3, wherein the at least one drum (130) has a shape selected from the group consisting of oval, circular, and any of the polygons.

5. The adjustable accumulator of Claim 3, wherein said drum (130) does not surround the entire outer tube (60).

6. The adjustable accumulator of Claim 3, wherein
said drum (130) further comprises at least one partition (170) to separate said interior of the drum into at least two compartments (180); and wherein
said compartment (180) has a compartment interior and said volume comprises said compartment interior when said inner tube (50) is adjusted to a position so that the compartment interior is in fluid communication with said volume.

7. The adjustable accumulator of Claim 6, wherein at least one compartment (180) is detachable from the adjustable accumulator.

8. The adjustable accumulator of Claim 6, wherein at least two compartments (180) are substantially identical in size and shape.

9. The adjustable accumulator of Claim 6, wherein the partition (170) is downwardly sloped toward to the inner tube (50).

10. The adjustable accumulator of Claim 1, wherein said chemical comprises gas, liquid, solid, or any combination thereof.

11. The adjustable accumulator of Claim 1, further comprising at least one O-ring (230) to retain the chemical in the adjustable accumulator.

12. The adjustable accumulator of Claim 1, further comprising at least one adjustable retainer (190, 210, 220) that holds the inner tube (50) and the outer tube (60) at a desired position.

13. The adjustable accumulator of Claim 12, wherein the retainer is a setscrew (190).

14. The adjustable accumulator of Claim 12, wherein the retainer is a spacer (210).

15. The adjustable accumulator of Claim 12, wherein the retainer is a shaft or jack (220).

16. A system for sterilizing equipment, said system comprising:
an adjustable accumulator (20) according to Claim 1, in which said chemical is a germicide;
a germicide reservoir (24) in fluid communication with said adjustable accumulator (20) so as to deliver said germicide to said adjustable accumulator (20); and
a sterilization chamber (10) in fluid communication with said adjustable accumulator (20) so as to receive said germicide from said adjustable accumulator (20).

17. The system of Claim 16, wherein
the outer tube (60) further comprises at least one drum (130) extending from said outer tube (60); wherein
said drum (130) has an interior; wherein
said volume comprises said interior when said inner tube (50) is adjusted to a position so that the interior of said drum (130) is in fluid communication with said volume.

18. The system of Claim 17, wherein
said drum (130) further comprises at least one partition (170) to separate said interior of the drum into at least two compartments (180); wherein
said compartment (180) has a compartment interior and said volume comprises said compartment interior when said inner tube (50) is adjusted to a position so that the compartment interior is in fluid communication with said volume.

19. The system of Claim 16, further comprising a vaporizer (28), wherein
said vaporizer (28) is in fluid communication with said adjustable accumulator (20) via said second valve (32) and is in fluid communication with said sterilization chamber (10).

20. The system of Claim 16, further comprising a vacuum pump (36) in fluid communication with said sterilization chamber (10).

21. The system of Claim 16, further comprising a source of plasma.

22. The system of Claim 16, wherein said first valve (30) is located between said adjustable accumulator and said reservoir and said second valve (32) is located between said adjustable accumulator (20) and said sterilization chamber (10).

23. The system of Claim 16, wherein said germicide comprises hydrogen peroxide.

24. A method for sterilizing an article in a chamber (10), said method comprising:
placing said article in said chamber (10);
delivering a volume of germicide from a reservoir (24) containing germicide to said chamber (10) via an adjustable accumulator (20) according to Claim 1 in which said chemical is said germicide; and
contacting said germicide with said article; thereby
sterilizing said article in said chamber (10).

25. The method of Claim 24, further comprising evacuating said chamber (10).

26. The method of Claim 24, further comprising vaporizing said germicide.

27. The method of Claim 24, wherein
the outer tube (60) further comprises at least one drum (13) extending from said outer tube (60); wherein
said drum (130) has an interior; and wherein
said volume comprises said interior when said inner tube (50) is adjusted to a position so that the interior of said drum (130) is in fluid communication with said volume.

28. The method of Claim 24, wherein
said drum (130) further comprises at least one partition (170) to separate said interior of the drum into at least two compartments (180); wherein
said compartment (180) has a compartment interior and said volume comprises said compartment interior when said inner tube (50) is adjusted to a position so that the interior of said compartment (180) is in fluid communication with said volume.

29. The method of Claim 24, wherein said germicide comprises hydrogen peroxide.

30. The method of Claim 24, further comprising opening and/or closing said first valve (30) between said adjustable accumulator (20) and said reservoir (24).

31. The method of Claim 24, further comprising opening and/or closing said second valve (32) between said adjustable accumulator (20) and said chamber (10).

32. The method of Claim 24, further comprising moving said inner tube (50) and/or said outer tube (60) so as to adjust the volume of the adjustable accumulator (20).

33. The method of Claim 26, wherein the vaporizing step comprises opening said second valve (32).

## Patentansprüche

1. Einstellbarer Speicher (20) mit einem Volumen zur Abgabe einer chemischen Substanz, dieser einstellbare Speicher umfaßt:
eine hohle innere Röhre (50), die ein erstes offenes Ende (70) und ein zweites Ende (90) verbunden mit einem ersten Ventil (30) aufweist; wobei
eine hohle äußere Röhre (60), die ein erstes offenes Ende (100) und ein zweites Ende (120) verbunden mit einem zweiten Ventil (32) aufweist; und
das erste offene Ende (70) der inneren Röhre (50) dazu geeignet ist, daß es in das erste offene Ende (100) der äußeren Röhre (60) hinein passt und dicht abschließt, und dadurch eine durch die äußere Röhre (60), die innere Röhre (50), das erste Ventil (30) und das zweite Ventil (32) begrenzte Kammer schafft, um die chemische Substanz zu speichern; wobei
das Volumen das Volumen des Raums ist, der von der Kammer eingeschlossen ist; und
wobei die innere Röhre (50) innerhalb der äußeren Röhre (60) zur Einstellung des Volumens der Kammer bewegbar ist.

2. Einstellbarer Speicher nach Anspruch 1, **dadurch gekennzeichnet, daß**
die äußere Röhre (60) und die innere Röhre (50) zylinderförmig sind.

3. Einstellbarer Speicher nach Anspruch 1, **dadurch gekennzeichnet, daß**
die äußere Röhre (60) ferner mindestens eine sich von der äußeren Röhre (60) erstreckende Trommel (130) umfaßt; wobei
die Trommel (130) ein Inneres aufweist, und wobei
das Volumen das Innere umfaßt, wenn die innere Röhre (50) so in eine Position eingestellt wird, daß das Innere der Trommel (130) sich mit dem Volumen in Flüssigkeitsverbindung befindet.

4. Einstellbarer Speicher nach Anspruch 3, **dadurch gekennzeichnet, daß**
die mindestens eine Trommel (130) eine ovale, eine kreisförmige und/oder irgendeine polygonale Form aufweist.

5. Einstellbarer Speicher nach Anspruch 3, **dadurch gekennzeichnet, daß**
die Trommel (130) nicht die gesamte äußere Röhre (60) umgibt.

6. Einstellbarer Speicher nach Anspruch 3, **dadurch gekennzeichnet, daß**
die Trommel (130) ferner mindestens eine Unterteilung (170) umfaßt, um das Innere der Trommel in mindestens zwei Sektionen (180) aufzuteilen; und wobei
die Sektion (180) ein Sektionsinneres aufweist und das Volumen das Sektionsinnere umfaßt, wenn die innere Röhre (50) so in eine Position eingestellt wird, daß das Sektionsinnere sich mit dem Volumen in Flüssigkeitsverbindung befindet.

7. Einstellbarer Speicher nach Anspruch 6, **dadurch gekennzeichnet, daß**
mindestens eine Sektion (180) von dem einstellbaren Speicher abtrennbar ist.

8. Einstellbarer Speicher nach Anspruch 6, **dadurch gekennzeichnet, daß**
mindestens zwei Sektionen (180) in Größe und Form im Wesentlichen identisch sind.

9. Einstellbarer Speicher nach Anspruch 6, **dadurch gekennzeichnet, daß**
die Unterteilung (170) in Richtung der inneren Röhre (50) nach unten geneigt ist.

10. Einstellbarer Speicher nach Anspruch 1, **dadurch gekennzeichnet, daß**
die chemische Substanz Gas, Flüssigkeit und/oder Feststoff, oder jedwede Kombination daraus umfaßt.

11. Einstellbarer Speicher nach Anspruch 1, **dadurch gekennzeichnet, daß**
der Speicher ferner mindestens einen O-Ring (230) umfaßt, um die chemische Substanz in dem einstellbaren Speicher zu speichern.

12. Einstellbarer Speicher nach Anspruch 1, **dadurch gekennzeichnet, daß**
der Speicher ferner mindestens eine einstellbare Halterung (190, 210, 220) umfaßt, die die innere Röhre (50) und die äußere Röhre (60) in der gewünschten Position hält.

13. Einstellbarer Speicher nach Anspruch 12, **dadurch gekennzeichnet, daß**
die Halterung eine Stellschraube (190) ist.

14. Einstellbarer Speicher nach Anspruch 12, **dadurch gekennzeichnet, daß**
die Halterung ein Abstandshalter (210) ist.

15. Einstellbarer Speicher nach Anspruch 12, **dadurch gekennzeichnet, daß**
die Halterung eine Welle oder eine Buchse (220) ist.

16. System zur Sterilisation von Ausrüstungsgegenständen, wobei das System umfaßt:
einen einstellbaren Speicher (20) nach Anspruch 1, bei dem die chemische Substanz ein Germizid ist;
ein Germizidreservoir (24) in Flüssigkeitsverbindung mit dem einstellbaren Speicher (20), um das Germizid an den einstellbaren Speicher (20) abzugeben; und
eine Sterilisations-Kammer (10) in Flüssigkeitsverbindung mit dem einstellbaren Speicher (20), um das Germizid von dem einstellbaren Speicher (20) zu empfangen.

17. System nach Anspruch 16, **dadurch gekennzeichnet, daß**
die äußere Röhre (60) ferner mindestens eine Trommel (130) umfaßt, die sich von der äußeren Röhre (60) erstreckt; wobei
die Trommel (130) ein Inneres besitzt; und
das Volumen das Innere umfaßt, wenn die innere Röhre (50) so in eine Position eingestellt wird, daß das Innere der Trommel (130) sich mit dem Volumen in Flüssigkeitsverbindung befindet.

18. Das System nach Anspruch 17, **dadurch gekennzeichnet, daß**
die Trommel (130) ferner mindestens eine Unterteilung (170) umfaßt, um das Innere der Trommel in mindestens zwei Sektionen (180) aufzuteilen; wobei
die Sektion (180) ein Sektionsinneres aufweist und das Volumen das Sektionsinnere umfaßt, wenn die innere Röhre (50) in eine Position so eingestellt wird, daß das Sektionsinnere sich mit dem Volumen in Flüssigkeitsverbindung befindet.

19. System nach Anspruch 16, **dadurch gekennzeichnet, daß**
das System ferner einen Zerstäuber (28) umfaßt, wobei sich der Zerstäuber (28) mittels des zweiten Ventils (32) in Flüssigkeitsverbindung mit dem einstellbaren Speicher (20) und sich in Flüssigkeitsverbindung mit der Sterilisations-Kammer (10) befindet.

20. System nach Anspruch 16, **dadurch gekennzeichnet, daß**
das System ferner eine Vakuumpumpe (36), die sich mit der Sterilisations-Kammer (10) mit der Sterilisations-Kammer (10) in Flüssigkeitsverbindung befindet, umfaßt.

21. System nach Anspruch 16, **dadurch gekennzeichnet, daß**
das System ferner eine Plasmaquelle umfaßt.

22. System nach Anspruch 16, **dadurch gekennzeichnet, daß**
das erste Ventil (30) zwischen dem einstellbaren Speicher und dem Reservoir angeordnet ist und das zweite Ventil (32) zwischen dem einstellbaren Speicher (20) und der Sterilisations-Kammer (10) angeordnet ist.

23. System nach Anspruch 16, **dadurch gekennzeichnet, daß**
das Germizid Wasserstoffperoxid umfaßt.

24. Verfahren zur Sterilisation eines Gegenstands in einer Kammer (10), wobei das Verfahren umfaßt:
das Einbringen des Gegenstands in die Kammer (10);
das Abgeben eines Volumens an Germizid aus einem Germizid enthaltenden Reservoir (24) an die Kammer (10) mittels eines einstellbaren Speichers (20) nach Anspruch 1, in dem die chemische Substanz ein Germizid ist; und
das In-Kontakt-Bringen des Germizides mit dem Gegenstand; dadurch
das Sterilisieren des Gegenstands in der Kammer (10).

25. Verfahren nach Anspruch 24, **dadurch gekennzeichnet, daß**
das Verfahren ferner das Evakuieren der Kammer (10) umfaßt.

26. Verfahren nach Anspruch 24, **dadurch gekennzeichnet, daß**
das Verfahren ferner das Zerstäuben des Germizides umfaßt.

27. Verfahren nach Anspruch 24, **dadurch gekennzeichnet, daß**
die äußere Röhre (60) ferner mindestens eine Trommel (130) umfaßt, die sich von der äußeren Röhre (60) erstreckt; wobei
die Trommel (130) ein Inneres aufweist; und wobei
das Volumen das Innere umfaßt, wenn die innere Röhre (50) so in eine Position eingestellt wird, daß das Innere der Trommel (130) sich mit dem Volumen in Flüssigkeitsverbindung befindet.

28. Verfahren nach Anspruch 24, **dadurch gekennzeichnet, daß**
die Trommel (130) femermindestens eine Unterteilung (170) umfaßt, um das Innere der Trommel in mindestens zwei Sektionen (180) aufzuteilen; wobei
die Sektion (180) ein Sektionsinneres aufweist und das Volumen das Sektionsinnere umfaßt, wenn die innere Röhre (50) so in eine Position eingestellt wird, daß das Innere der Sektion (180) sich mit dem Volumen in Flüssigkeitsverbindung befindet.

29. Verfahren nach Anspruch 24, **dadurch gekennzeichnet, daß**
das Germizid Wasserstoffperoxid enthält.

30. Verfahren nach Anspruch 24, **dadurch gekennzeichnet, daß**
das Verfahren ferner das Öffnen und/oder Schließen des ersten Ventils (30) zwischen dem einstellbaren Speicher (20) und dem Reservoir (24) umfaßt.

31. Das Verfahren nach Anspruch 24, **dadurch gekennzeichnet, daß**
das Verfahren ferner das Öffnen und/oder Schließen des zweiten Ventils (32) zwischen dem einstellbaren Speicher (20) und der Kammer (10) umfaßt.

32. Verfahren nach Anspruch 24, **dadurch gekennzeichnet, daß**
das Verfahren ferner das Bewegen der inneren Röhre (50) und/oder der äußeren Röhre (60) umfaßt, um das Volumen des einstellbaren Speichers (20) einzustellen.

33. Verfahren nach Anspruch 26, **dadurch gekennzeichnet, daß**
der Zerstäubungsschritt das Öffnen des zweiten Ventils (32) umfaßt.

## Revendications

1. Accumulateur réglable (20) ayant un volume destiné à fournir un produit chimique, ledit accumulateur réglable comprenant :
un tube interne creux (50) ayant une première extrémité ouverte (70) et une seconde extrémité (90) reliée à une première valve (30) ; et
un tube externe creux (60) ayant une première extrémité ouverte (100) et une seconde extrémité (120) reliée à une seconde valve (32) ; et
ladite première extrémité ouverte (70) du tube interne (50) est adaptée pour s'ajuster dans et hermétiquement avec la première extrémité ouverte (100) du tube externe (60), de façon à créer une chambre délimitée par le tube externe (60), le tube interne (50), la première valve (30) et la seconde valve (32), de manière à retenir ledit produit chimique ; dans lequel
ledit volume est le volume de l'espace enfermé par ladite chambre ; et dans lequel
le tube interne (50) est mobile à l'intérieur du tube externe (60) de façon à régler ledit volume de ladite chambre.

2. Accumulateur réglable selon la revendication 1, dans lequel le tube externe (60) et le tube interne (50) sont cylindriques.

3. Accumulateur réglable selon la revendication 1, dans lequel
le tube externe (60) comprend, en outre, au moins un tambour (130) s'étendant à partir dudit tube externe (60) ; dans lequel
ledit tambour (130) possède un intérieur ; et dans lequel
ledit volume comprend ledit intérieur lorsque ledit tube interne (50) est réglé dans une position telle que l'intérieur dudit tambour (130) est en communication fluide avec ledit volume.

4. Accumulateur réglable selon la revendication 3, dans lequel l'au moins un tambour (130) a une forme choisie dans le groupe constitué des formes ovale, circulaire, et d'un polygone quelconque.

5. Accumulateur réglable selon la revendication 3, dans lequel ledit tambour (130) n'entoure pas la totalité du tube externe (60).

6. Accumulateur réglable selon la revendication 3, dans lequel
ledit tambour (130) comprend, en outre, au moins un cloisonnage (170) destiné à séparer ledit intérieur du tambour en au moins deux compartiments (180) ; et dans lequel
ledit compartiment (180) comporte un intérieur de compartiment et ledit volume comprend ledit intérieur de compartiment lorsque ledit tube interne (50) est réglé dans une position telle que l'intérieur du compartiment est en communication fluide avec ledit volume.

7. Accumulateur réglable selon la revendication 6, dans lequel au moins un compartiment (180) est détachable de l'accumulateur réglable.

8. Accumulateur réglable selon la revendication 6, dans lequel au moins deux compartiments (180) sont sensiblement de tailles et de formes identiques.

9. Accumulateur réglable selon la revendication 6, dans lequel le cloisonnage (170) est incliné vers le bas vers le tube interne (50).

10. Accumulateur réglable selon la revendication 1, dans lequel ledit produit chimique comprend un gaz, un liquide, un solide ou une combinaison quelconque de ceux-ci.

11. Accumulateur réglable selon la revendication 1, comprenant, en outre, au moins un joint torique (230) destiné à retenir le produit chimique dans l'accumulateur réglable.

12. Accumulateur réglable selon la revendication 1, comprenant, en outre, au moins un dispositif de retenue réglable (190, 210, 220) qui maintient le tube interne (50) et le tube externe (60) dans une position désirée.

13. Accumulateur réglable selon la revendication 12, dans lequel le dispositif de retenue est une vis de réglage (190).

14. Accumulateur réglable selon la revendication 12, dans lequel le dispositif de retenue est une pièce d'écartement (210).

15. Accumulateur réglable selon la revendication 12, dans lequel le dispositif de retenue est un arbre ou un vérin (220).

16. Système pour stériliser un équipement, ledit système comprenant :
un accumulateur réglable (20) selon la revendication 1, dans lequel ledit produit chimique est un germicide ;
un réservoir de germicide (24) en communication fluide avec ledit accumulateur réglable (20) de façon à fournir ledit germicide audit accumulateur réglable (20) ; et
une chambre de stérilisation (10) en communication fluide avec ledit accumulateur réglable (20) de façon à recevoir ledit germicide depuis ledit accumulateur réglable (20).

17. Système selon la revendication 16, dans lequel
le tube externe (60) comprend, en outre, au moins un tambour (130) s'étendant à partir dudit tube externe (60) ; dans lequel
ledit tambour (130) possède un intérieur ; et dans lequel
ledit volume comprend ledit intérieur lorsque ledit tube interne (50) est réglé dans une position telle que l'intérieur dudit tambour (130) est en communication fluide avec ledit volume.

18. Système selon la revendication 17, dans lequel
ledit tambour (130) comprend, en outre, au moins un cloisonnage (170) destiné à séparer ledit intérieur du tambour en au moins deux compartiments (180) ; dans lequel
ledit compartiment (180) comporte un intérieur de compartiment et ledit volume comprend ledit intérieur de compartiment lorsque ledit tube interne (50) est réglé dans une position telle que l'intérieur du compartiment est en communication fluide avec ledit volume.

19. Système selon la revendication 16, comprenant, en outre, un vaporisateur (28), dans lequel
ledit vaporisateur (28) est en communication fluide avec ledit accumulateur réglable (20) par l'intermédiaire de ladite seconde valve (32) et est en communication fluide avec ladite chambre de stérilisation (10).

20. Système selon la revendication 16, comprenant, en outre, une pompe à vide (36) en communication fluide avec ladite chambre de stérilisation (10).

21. Système selon la revendication 16, comprenant, en outre, une source de plasma.

22. Système selon la revendication 16, dans lequel ladite première valve (30) est disposée entre ledit accumulateur réglable et ledit réservoir et ladite seconde valve (32) est disposée entre ledit accumulateur réglable (20) et ladite chambre de stérilisation (10).

23. Système selon la revendication 16, dans lequel ledit germicide comprend du peroxyde d'hydrogène.

24. Procédé pour stériliser un objet dans une chambre (10), ledit procédé comprenant :
le placement dudit objet dans ladite chambre (10) ;
la fourniture à la ladite chambre (10) d'un certain volume de germicide depuis un réservoir (24) contenant un germicide par l'intermédiaire d'un accumulateur réglable (20) selon la revendication 1, dans lequel ledit produit chimique est ledit germicide ; et
la mise en contact dudit germicide avec ledit objet ; de façon à
stériliser ledit objet dans ladite chambre (10).

25. Procédé selon la revendication 24, comprenant, en outre, le vidage de ladite chambre (10).

26. Procédé selon la revendication 24, comprenant, en outre, la vaporisation dudit germicide.

27. Procédé selon la revendication 24, dans lequel
le tube externe (60) comprend, en outre, au moins un tambour (13) s'étendant à partir dudit tube externe (60) ; dans lequel
ledit tambour (130) possède un intérieur ; et dans lequel
ledit volume comprend ledit intérieur lorsque ledit tube interne (50) est réglé dans une position telle que l'intérieur dudit tambour (130) est en communication fluide avec ledit volume.

28. Procédé selon la revendication 24, dans lequel
ledit tambour (130) comprend, en outre, au moins un cloisonnage (170) destiné à séparer ledit intérieur du tambour en au moins deux compartiments (180) ; et dans lequel
ledit compartiment (180) comporte un intérieur de compartiment et ledit volume comprend ledit intérieur de compartiment lorsque ledit tube interne (50) est réglé dans une position telle que l'intérieur dudit compartiment est en communication fluide avec ledit volume.

29. Procédé selon la revendication 24, dans lequel ledit germicide comprend du peroxyde d'hydrogène

30. Procédé selon la revendication 24, comprenant, en outre, l'ouverture et/ou la fermeture de ladite première valve (30) entre ledit accumulateur réglable (20) et ledit réservoir (24).

31. Procédé selon la revendication 24, comprenant, en outre, l'ouverture et/ou la fermeture de ladite seconde valve (32) entre ledit accumulateur réglable (20) et ladite chambre (10).

32. Procédé selon la revendication 24, comprenant, en outre, le déplacement dudit tube interne (50) et/ou dudit tube externe (60) de façon à régler le volume de l'accumulateur réglable (20).

33. Procédé selon la revendication 26, dans lequel l'étape de vaporisation comprend l'ouverture de ladite seconde valve (32).
